(19) Europäisches Patentamt
European Patent Office
Office européen des brevets

(11) Publication number: **0 269 258**
**A2**

(12) **EUROPEAN PATENT APPLICATION**

(21) Application number: 87309475.9

(22) Date of filing: 27.10.87

(51) Int. Cl.[4]: C07D 207/12 , C07C 121/453 , C07C 121/86 , C07C 125/065

(30) Priority: 27.10.86 US 923544

(43) Date of publication of application: 01.06.88 Bulletin 88/22

(84) Designated Contracting States: AT BE CH DE ES FR GB GR IT LI LU NL SE

(71) Applicant: A.H. ROBINS COMPANY, INCORPORATED
1405 Cummings Drive PO Box 26609
Richmond Virginia 23261-6609(US)

(72) Inventor: Lo, Young Sek
200 Tamarack Road
Richmond Virginia 23229(US)

(74) Representative: Sheard, Andrew Gregory et al
Kilburn & Strode 30, John Street
London WC1N 2DD(GB)

(54) **Process for the preparation of a pyrrolidinol compound.**

(57) In a process for the preparation of a pyrrolidinol compound having the general formula I, a hydroxybutyronitrile compound having a general formula II undergoes reductive cyclisation, in an acid containing solution, with hydrogen gas in the presence of a Raney nickel catalyst.

EP 0 269 258 A2

Formula I

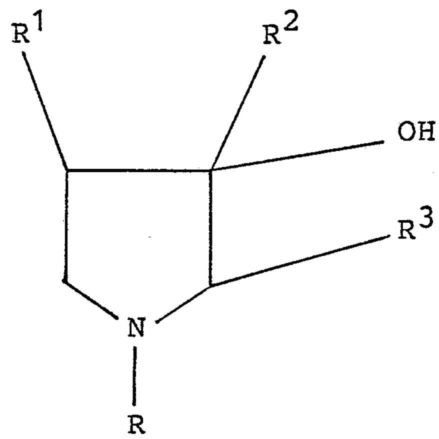

Formula II

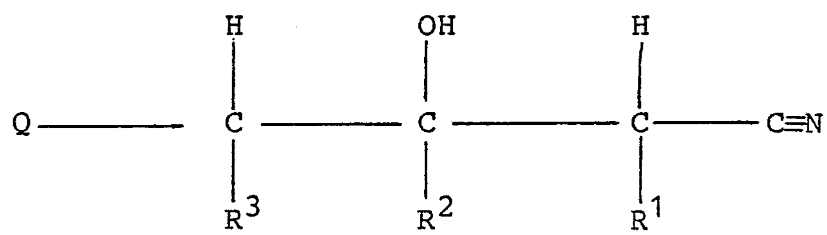

R represents a hydrogen atom, a loweralkyl group, a loweralkenyl group, a cycloalkyl group, a cycloalkyl-loweralkyl group, a phenyl-loweralkyl group and a substituted phenyl-loweralkyl group; and $R^1$, $R^2$ and $R^3$ independently represent a hydrogen atom, a loweralkyl group and a loweralkenyl group.

Novel hydroxybutyronitrile compounds and a process for the preparation of a hydroxybutyronitrile compound is also disclosed.

# PROCESS FOR THE PREPARATION OF PYRROLIDINOL COMPOUND

The present invention relates to a process for the preparation of a pyrrolidinol compound, to a hydroxybutyronitrile compound and its use as in intermediate in the preparation of the pyrrolidinol compound and a process for the preparation of a hydroxybutyronitrile compound.

To date, several routes to pyrrolidinols have been available for use as described in US patents 2,838,521 and 2,882, 276 and in Synthetic Communications 13 (13) 1117-1123 (1983).

These methods include:

1) Commercial conversion of 1, 2, 4-butanetriol to 1, 4-dibromo-2-butanol with hydrogen bromide at 140°C followed by condensation with a secondary amine. Debenzylation of N-benzyl-3-pyrrolidinol so produced with hydrogen over palladium on carbon leads to 3-pyrrolidinol;

2) Reduction of N-substituted-3-pyrrolidinones with lithium aluminium hydride;

3) Heating cis 1,4-dichloro-2-butenes with benzylamine to produce N-benzyl-3-pyrrolidinol and debenzylating as in 1) above; and

4) Heating malic acid with benzylamine in ethanol at 170°C to give N-benzyl-3-hydroxysuccinimide and reducing with lithium aluminium hydride to give N-benzyl-3-pyrrolidinol.

Japanese patent 32766 (1985) discloses reductive cyclisation of 2-cyanoethylglycine ethyl ester using palladium on carbon catalyst and hydrogen at 50°C and 10 kg/cm$^2$ pressure for 3 hours. In contrast, in the present invention a different class of reactant and product are involved, the end product being a 3-pyrrolidinol and in further contrast, hydrogenation over palladium on carbon catalyst alone did not produce the cyclisation brought about by Raney nickel catalyst in the present invention.

One chemical intermediate useful in the process of the present invention; namely, 4-amino-3-hydroxybutyronitrile is a known compound as reported by Jung, M.E. in J. AMER. CHEM. SOC. 102, 6304 (1980). The carbonate of this compound has been used as an intermediate for the manufacture of alpha-amino-beta-hydroxybutyric acid as disclosed in JAP. 16,504 (66) (C.A. 66, 18309v). 4-Azido-3-hydroxybutyronitrile also useful in the process is known.

British Patent 913,856 (C.A. 59, 1787) describes the preparation of 4-(N-methyl-N-phenylamino)-3-hydroxybutyronitrile from 4-(methyl-N-phenyl)-3-hydroxybutyl chloride and sodium cyanide in ethanol. The compound is useful in dye preparation. The foregoing anilino derivative is not useful in the process of the present invention as it cannot be reductively cyclised in the process, nor can the phenol radical be removed as can the benzyl radical.

The use of N,N-diethylamino-3-hydroxybutyronitrile in the preparation of high molecular weight therapeutic agents has been described in IOWA STATE COLL. J. SCI. 21, 41-45 (1946) (C.A. 41, 3044b). The compound is not useful in the process of the present invention as it cannot be cyclised thereby.

Compounds prepared by the process of the present invention are useful in the preparation of pharmaceuticals as described in the following US patents:

2,838,521
2,830,997
2,956,062
3,301,869
2,882,276
and 4,592,866

The first aspect of the present invention relates to a process for the preparation of pyrrolidinols, which may in practice be found to be economical.

Thus, according to a first aspect of the present invention, there is provided a process for the preparation of a pyrrolidinol compound having a general formula I or a salt thereof, the process comprising reductive cyclisation of a hydroxybutyronitrile compound having a general formula II or a salt thereof, in an acid containing solution, with hydrogen gas, in the presence of a catalyst comprising Raney nickel:

Formula I

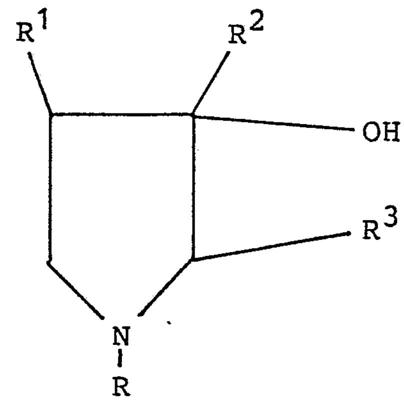

Formula II

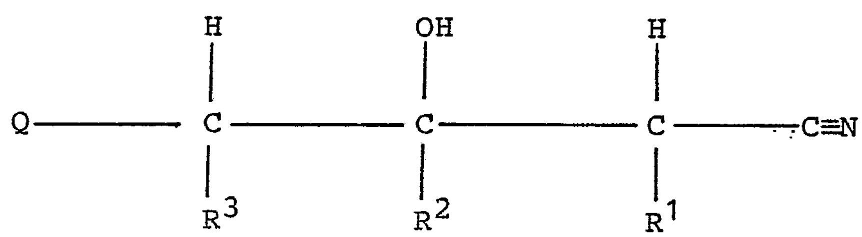

where R represents
a hydrogen atom,
a lower alkyl group ($C_1$ to $C_8$)
a lower alkenyl group ($C_2$ to $C_8$)
a cycloalkyl group ($C_3$ to $C_9$)
a cycloalkyl-loweralkyl group ($C_4$ to $C_{13}$)
a phenyl-lower alkyl group ($C_7$ to $C_{14}$),
or
a $(Y)_{1-3}$-phenyl-lower alkyl group ($C_7$ to $C_{14}$);
and
Y represents
a lower alkyl group ($C_1$ to $C_8$),
a lower alkoxy group ($C_1$ to $C_8$),
a halogen atom
or
a trifluoromethyl group;
and each of $R^1$, $R^2$ and $R^3$ independently represents

a hydrogen atom,
a lower alkyl group ($C_1$ to $C_8$)
or
a lower alkenyl group ($C_2$ to $C_8$);
and where Q represents a group R-(NH)-or an azido group.

The pyrrolidinol compound is optionally substituted in the 1, 2, 3 and/or 4 positions.

Preferably, the process further comprises separation of the catalyst from the pyrrolidinol compound which is in an acid containing solution.

The process optionally further comprises substantial evaporation of a solvent from the acid containing solution, addition of a high boiling diluent, neutralisation of the acid addition salt with a base, and vacuum

distillation to produce a distillate comprising the pyrrolidinol compound as a free base.

An optional feature of the process is that the catalyst comprises a mixture of a Raney nickel catalyst and a palladium on carbon catalyst.

The pyrrolidinol compound may be a racemic mixture or one or more resolved enantiomers.

The pyrrolidinol compound may optionally be substantially in either the 3-R or the 3-S enantiomeric form.

The acid containing solution preferably comprises a non-interfering acid. It is a preferred feature that the hydroxybutyronitrile compound comprises an acid addition salt of the compound of general formula II.

The acid is optionally supplied by hydrochloric acid.

The pH of the solution during reductive cyclisation is preferred to be in the range from 5 to 7.

A preferred solvent is an isopropanol-water mixture, and this most advantageously has a volume percent of isopropanol in the range from 70 to 90.

Preferred pyrrolidinol compounds which are preparable by a process according to the first aspect of the present invention are those in which R represents lower ($C_1$ -$C_8$) alkyl, such as N-methyl-and N-ethyl-3-pyrrolidinols. Especially preferred hydroxy butyronitrile and pyrrolidinol compounds include the following:

4-(N-ethylamino)-3-hydroxybutyronitrile [for the preparation of N-ethyl-3-pyrrolidinol],

4-(N-methylamino)-3-hydroxybutyronitrile [for the preparation of N-methyl-3-pyrrolidinol],

4-amino-3-hydroxybutyronitrile [for the preparation of 3-pyrrolidinol],

4-azido-3-hydroxybutyronitrile [for the preparation of 3-pyrrolidinol],

4-methylamino-3-hydroxybutyronitrile [for the preparation of N-methyl-3-pyrrolidinol ],

4-ethylamino-3-hydroxybutyronitrile [for the preparation of N-ethyl-3-pyrrolidinol],

4-(N-methylamino)-3-hydroxy-3-methyl-butyronitrile [for the preparation of N,3-Dimethyl-3-pyrrolidinol],

4-(N-methylamino)-3-hydroxy-2-methyl-butyronitrile [for the preparation of N,4-Dimethyl-3-pyrrolidinol],

or their salts.

A particularly preferred hydroxybutyronitrile compound has the general formula IIa or a salt thereof:

Formula IIa

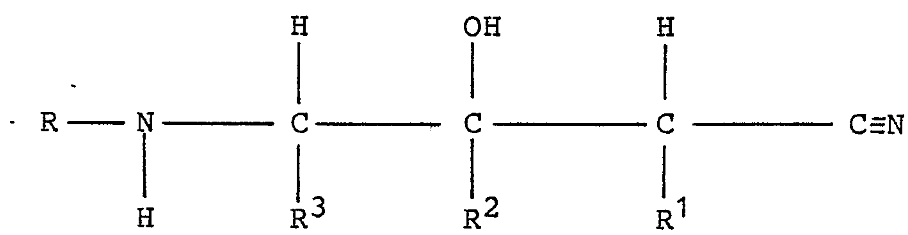

where R does not represent hydrogen.

Hydroxybutyronitrile compounds may be derived from a precursor having the general formula III or a salt thereof:

Formula III

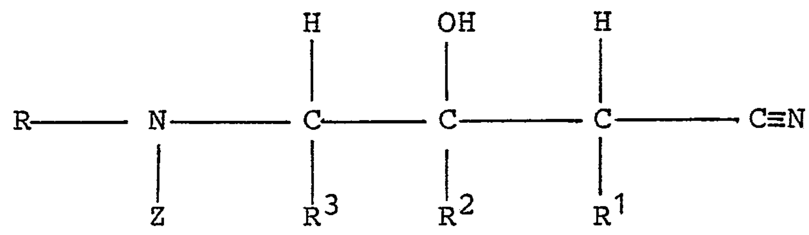

where Z represents an amine protecting group.

Preferred amine protecting groups are as follows:

Z represents
a benzyl group
an alpha-methylbenzyl group
an diphenylmethyl group
an alpha-phenylmethyl group
a benzyloxycarbonyl group
a diphenylmethoxycarbonyl group
a beta, beta, beta-trichloroethoxycarbonyl group
an isobutoxycarbonyl group
or
a t-butyloxycarbonyl group
with the proviso that R represents a hydrogen atom only when Z represents benzyl.

The acid addition salt of the compound of general formula I and the acid addition salt of the compound of general formula II are common forms in which the compounds exist at various stages of the process. In the first phase of the process, the compounds are generally present as acid addition salts in solution.

Compounds of Formula II other than wherein Q represents an azido group or an $NH_2$-group have not previously been reported in the prior art literature.

According to a second aspect of the present invention there is provided an hydroxybutyronitrile compound having the general formula IV or a salt thereof:

Formula IV

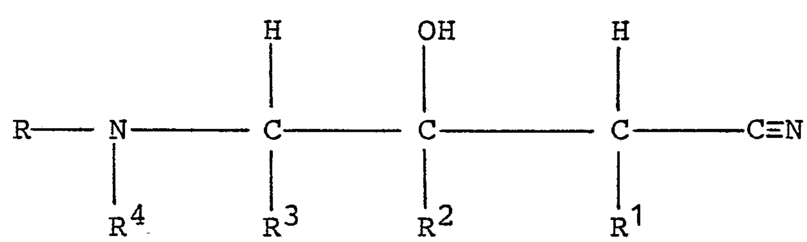

where R represents
a hydrogen atom
a lower alkyl group ($C_1$ to $C_8$)
a lower alkenyl group ($C_2$ to $C_8$)
a cycloalkyl group ($C_3$ to $C_9$)
a cycloalkyl-lower alkyl group ($C_4$ to $C_{13}$)
a phenyl-lower alkyl group ($C_7$ to $C_{14}$)
or
a $(Y)_{1\text{-}3}$-phenyl-lower alkyl group ($C_7$ to $C_{14}$)
and
Y represents
a lower alkyl group ($C_1$ to $C_8$)

a lower alkoxy group ($C_1$ to $C_8$)
a halogen atom
or
a trifluoromethyl group
and each of
$R^1$, $R^2$ and $R^3$ independently represents

a hydrogen atom
a lower alkyl group ($C_1$ to $C_8$)
or
a lower alkenyl group ($C_2$ to $C_8$)

$R^4$ represents a hydrogen atom or Z

and Z represents an amine protecting radical

with the proviso that R represents a hydrogen atom only when $R^4$ represents Z, and Z represents a benzyl group.

Preferred Z groups include the following:

Z represents
benzyl group
an alpha-methylbenzyl group
a diphenylmethyl group
an alpha-phenylmethyl group
a benzyloxycarbonyl group
a diphenylmethoxycarbonyl group
a beta, beta, beta-trichloroethoxycarbonyl group
an isobutoxycarbonyl group
a phenylmethoxycarbonyl group
or
a t-butyloxycarbonyl group.

A preferred compound of general formula IV has $R^4$ representing Z and particularly preferred examples includes 4-(N-methyl-N-benzylamino)-3-hydroxybutyronitrile or a salt thereof, and 4-(N-ethyl-N-benzylamino)-3-hydroxybutyronitrile or a salt thereof.

Optionally, R does not represent hydrogen atom, and, in this case, $R^4$ optionally represents a hydrogen atom.

Particularly preferred examples of compounds of general formula IV further include:

4 - (N-ethylamino)-B-hydroxybutyronitrile
4 - (N-methylamino)-3-hydroxybutyronitrile
4 - amino-3-hydroxybutyronitrile
4 - azido-3-hydroxybutyronitrile
4 - methylamino-3-hydroxybutyronitrile
4 - ethylamino-3-hydroxybutyronitrile
4 (N-methylamino)-3-hydroxy-3-methylbutyronitrile
or 4 - (N-methylamino)-3-hydroxy-2-methylbutyronitrile
or a salt thereof.

Preferred compounds of general formula IV are of general formula IIa or are salts thereof:

Formula IIa

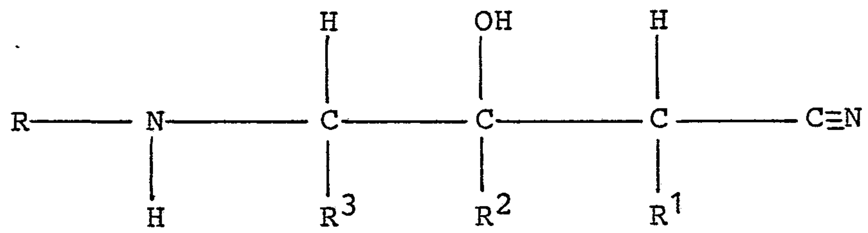

where R does not represent a hydrogen atom

Other preferred compounds of general formula IV are amine protected, and are of the general formula III or are salts thereof;

Formula III

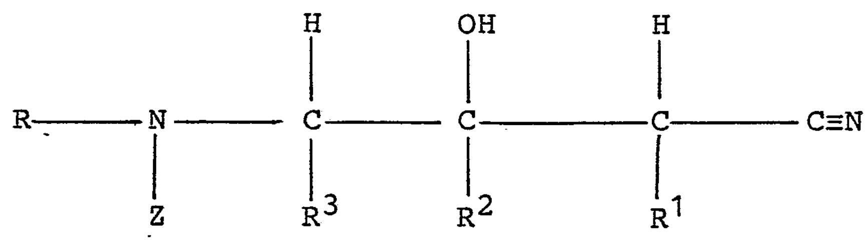

Compounds of Formula III may be used as precursors to Formula II compounds.

According to a third aspect of the present invention there is provided a process for the preparation of a compound having the general formula IV or a salt thereof:

<u>Formula IV</u>

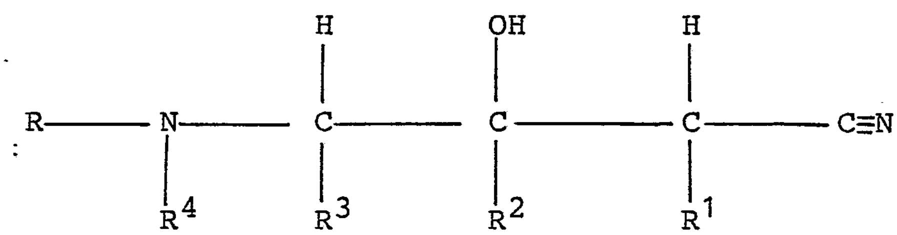

where R represents
a lower alkyl group ($C_1$ to $C_8$)
a lower alkenyl group ($C_2$ to $C_8$)
a cycloalkyl group ($C_3$ to $C_9$)
a cycloalkyl-lower alkyl group ($C_4$ to $C_{13}$)
a phenyl-lower alkyl group ($C_7$ to $C_{14}$)
or
a $(Y)_{1-3}$-phenyl-lower alkyl group ($C_7$ to $C_{14}$)
and
Y represents a lower alkyl group ($C_1$ to $C_8$)
a lower alkoxy group ($C_1$ to $C_8$)
a halogen atom
or a trifluoromethyl group

and each of

$R^1$, $R^2$ and $R^3$ independently represents
a hydrogen atom
a lower alkyl group ($C_2$ to $C_8$)
or
a lower alkenyl group ($C_2$ to $C_8$)

$R^4$ represents a hydrogen atom or Z

and Z represents an amine protecting radical

and, preferably,
Z represents
a benzyl group
an alpha-methylbenzyl group
a diphenylmethyl group
an alpha-phenylmethyl group
a benzyloxycarbonyl group
a diphenylmethoxycarbonyl group
a beta, beta, beta-trichloroethoxycarbonyl group
an isobutoxycarbonyl group
a phenylmethoxycarbonyl group
or
a t-butyloxycarbonyl group.

with the proviso that R represents a hydrogen atom only when Z represents benzyl,

wherein the process comprises reaction of a compound of formula VII with a metal cyanide

Formula VII

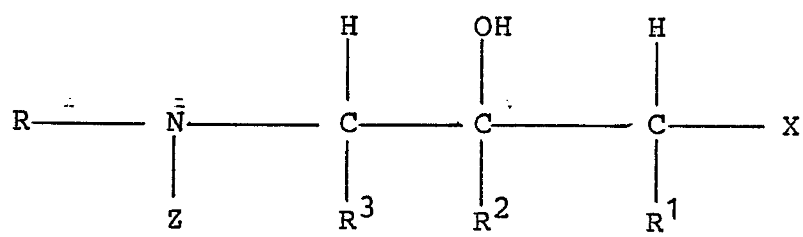

wherein X represents a reactive leaving group and $R^1$, $R^2$, $R^3$ and Z are as defined for general formula IV, and optiontally deprotecting the amine group.

A compound of general formula VII can be prepared by reacting a compound of general formula V

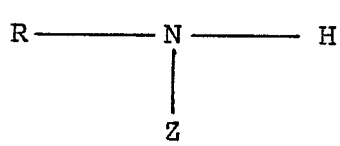

(V)

wherein R and Z are as defined for general formula IV with a compound of general formula VI

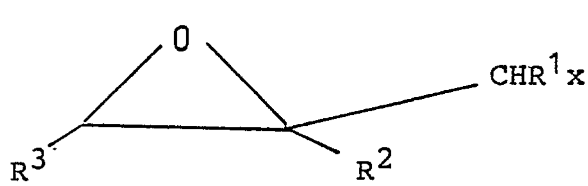

(VI)

wherein $R^1$, $R^2$ and $R^3$ are as defined for general formula IV and X represents a reactive leaving group.

Preferred leaving groups are halogen atoms and O-tosyl and 0-mesyl groups.

The invention can therefore be seen to relate to the production of 3-pyrrolidinols, the production of (generally N substituted) 4-amino-3-hydroxybutyronitriles and to certain novel 4-amino-3-hydroxybutyronitriles themselves.

In the further definition of symbols in the formulas hereof and where they appear elsewhere throughout this specification and in the claims, the terms have the following significance.

The term "lower alkyl" as used herein, unless otherwise specified, includes straight and branched chain radicals of up to eight carbons inclusive and is exemplified by such groups as methyl, ethyl, propyl, isopropyl, butyl, sec. butyl, tert. butyl, amyl, isoamyl, hexyl, heptyl, and octyl radicals and the like. The term "lower alkoxy" refers to -O-loweralkyl.

The term "lower alkenyl" as used herein refers to 2-8 carbon chain hydrocarbon radicals having a carbon-to-carbon double bond, including such as allyl and isobutenyl radicals.

The term "cycloalkyl" as used herein includes primarily cyclic alkyl radicals containing 3-9 carbon atoms inclusive and includes such groups as cyclopropyl, cyclobutyl, cyclopentyl, cyclohexyl, methylcyclohexyl, cycloheptyl and the like.

The terms "halo", "halide" or "halogen" when referred to herein include fluorine, chlorine, bromine, and iodine unless otherwise stated.

The term "non-interfering radicals" when used in conjunction with phenyl substitution is intended to mean a radical which does not interfere with any of the reactions including reductive cyclization process involving Raney nickel.

The term "reactive leaving group" refers to any of the conventional radicals such as halo, -o-tosyl or o-mesyl which may be used to introduce a cyano group by reacting with metal cyanide reagents.

The term "amine protecting group" refers to a radical which temporarily prevents unwanted reaction with the hydrogen of an amine function, such protecting group being removable in a subsequent step referred to as "deprotection." Representative of such amine protecting groups which suitably protect and can be suitably removed without disrupting the integrity of the 4-amino-3-hydroxybutyronitrile chemical intermediates involved include those of two general classes a) and b) as follows:

a) Urethane Derivatives. Examples of suitable urethane forming radicals which can be used for protection and the method of "deprotection" are as follows:

t-butoxycarbonyl (deprotect with acid),
isobutyloxycarbonyl (deprotect with ammonia and pyridine),
$\beta,\beta,\beta$-trichloroethoxycarbonyl (deprotect with zinc and acetic acid], or
benzyloxycarbonyl (deprotect with strong acid, e.g., HCl, HBr, HI or hydrogenation);

b) Benzyl Derivatives. Examples of suitable benzyl forming radicals which can be used for protection and the method of "deprotection" are as follows:

phenylmethyl (deprotect by hydrogenation over Pd/C),
$\alpha$-methylbenzyl (deprotect by hydrogenation over Pd/C), or
diphenylmethyl (deprotect by hydrogenation over Pd/C).

Reference is made to the textbook: "Protective Groups in Organic Chemistry by J. F. W. McOmie, publ. Plenum Press London & New York (1973), pp. 43-74", for discussion of protection and deprotection of primary and secondary amines.

In such amine protecting groups having a phenyl compound, the phenyl may be substituted by non-interfering radicals such as do not poison the Raney nickel catalyst or waste hydrogen gas by uptake thereof. Deprotection is accomplished by a suitable deprotecting agent as explained herein below.

The term "non-interfering acid" as used herein refers to an acid which does not poison the catalyst within the working range of pH 3-10.

The term "acid containing solution" specifies an acid has been added to the solution irrespective of the manner of addition, e.g., as solution, as a gas, or as acid addition salt of reactant, etc.

The term "reductive cyclisation" and "reductively cyclising" are terms referring to the act of reducing the nitrile group of a hydroxybutyronitrile and causing cyclisation to a pyrrolidinol.

A better understanding of the present invention and its may advantages will be had by referring to the following description and specific examples, given by way of illustration.

A process for preparing the 3-pyrrolidinols of Formula I from 4-azido or 4-amino-3-hydroxybutyronitriles is schematically illustrated by Chart I.

## CHART I

### Process for Preparation of 3-Pyrrolidinols

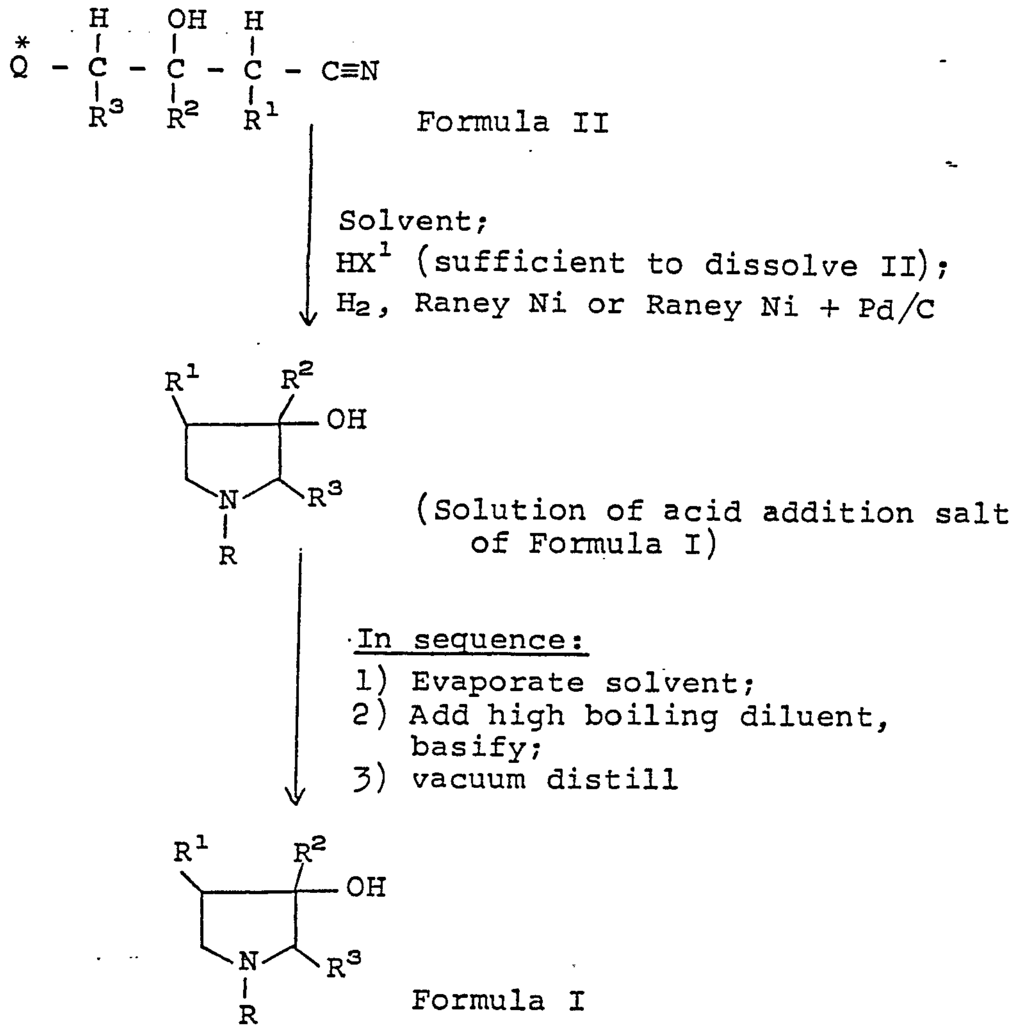


Footnote to Chart I:
*Q is $R-\underset{\substack{|\\ H}}{N}-$ or $N_3$ (azido), and R, $R^1$, $R^2$ and $R^3$ are as defined under Formula I; $HX^1$ is a non-interfering acid. Optical (R) and (S) isomers potentially available due to chiral center at the carbon bearing the hydroxyl group.

Briefly stated, a novel process for preparing the free base of the 3-pyrrolidinols of Formula I is comprised of the steps of:

Step 1, subjecting a hydroxybutyronitrile compound selected from the group having the formula:

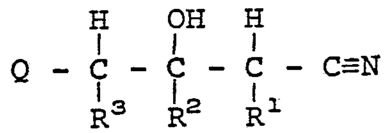

II

wherein $R^1$, $R^2$, and $R^3$ are as defined hereinabove under Formula I; Q is R- $\underset{H}{N}$ -
or $N_3$ (i.e, azido) and wherein R is as defined under Formula I and the optical isomers thereof in an acid containing solution having dissolved therein a non-interfering acid: $HX^1$, the acid optionally alternatively supplied by substituting an acid addition salt of compound II, to hydrogen gas under pressure in the presence of a catalyst comprised of Raney nickel or optionally a mixture of Raney nickel and palladium on carbon to give a slurry comprised of:

a) a solution having dissolved therein a pyrrolidinol compound having the formula

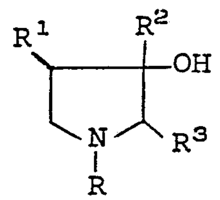

I

wherein R, $R^1$, $R^2$, and $R^3$ are as defined above, and

b) the catalyst in suspension and separating the catalyst from the slurry to give said pyrrolidinol compound in acid containing solution,

and optionally

Step 2, isolating the free base of the 3-pyrrolidinols of Formula I by the sequential procedure of

a) evaporating off the reaction solvent,

b) adding high boiling diluent and base, to neutralize the acid, giving the free base of the 3-pyrrolidinol, and,

c) vacuum distilling the free base of said pyrrolidinol compound.

The invention encompasses the process for the preparation of the acid containing solution of the pyrrolidinol compounds of Formula I of Step 1 by itself and the combination of Steps 1 and 2 together to give the free base pyrrolidinol compounds of Formula I.

The following general description is applicable to the foregoing process.

In the process, a compound of Formula II in a suitable solvent having an acidic component (Formula II compound as acid addition salt or added acid $HX^1$ if free base is used in amount sufficient to dissolve II), the solvent described as being capable of dissolving some water and preferably having water dissolved therein and the resulting solution having a pH of about 3-10, preferably about 5-7, is subjected to hydrogen gas under pressure at about 15-150 psi, preferably about 25-75 psi and about 0-100°C. temperature, preferably 20-50°C. in the presence of Raney nickel catalyst, optionally and preferably in the presence of Raney nickel and palladium on carbon catalysts until hydrogen uptake ceases and the compound of Formula II is substantially reductively cyclized to give a compound of Formula I as an acid salt in solution. The Formula I compound is then isolated by substantially removing the reaction solvent, adding a diluent carrier, neutralizing(or basifying) the acid salt and any excess $HX^1$ acid and vacuum distilling the Formula I free base.

The following more detailed description is also applicable to the process of the invention.

In Step 1, suitable solvents are those which will dissolve both the starting compound of Formula II and the product compound of Formula I, both in the presence of an acid, such acid being non-poisonous to the catalyst and in amount such that solubilization of compound II is attained and maintained, and the Raney nickel is not inactivated due to amount of acid. Suitable representative acids are mineral acids such as hydrochloric, hydrobromic, sulfuric or organic acids such as acetic acid or trifluoroacetic acid. Ratios of about 0.9 to 1.1 equivalents of acid per mole of compound II appear optimum and are therefore preferred. Above about 1.1 equivalent ratio of acid to compound II, Raney nickel is inactivated. As stated above, suitable solvents appear to have capacity to dissolve, at least, some water, although water may not be required in some solvents such as methanol or ethanol. However, it appears desirable to have at least a small amount of water present and as much as 100% water may be used. The use of too much water

increases difficulty in isolation of the product. Ratios of the lower alkanols to water in the ratio range of 70:30 to 90:10 volume % are preferred. Solvent mixtures comprised of isopropanol and water in the ratio range of 70:30 to 90:10 volume % are especially preferred. Suitable operating concentrations of starting compound II to liquid appear to be in the range of 5-25 weight %, preferably about 10 weight %.

Raney nickel is the required catalyst, palladium on carbon by itself being ineffective alone in the reductive cyclization. When the starting Formula II compound is derived by deprotecting the amino group of compound III by hydrogenation over palladium on carbon catalyst, it has been found preferable to conduct Step 1 of the process without separating the palladium on carbon catalyst as a higher yield was obtained when the palladium catalyst was left in. The preferred catalyst is a mixture of Raney nickel and palladium on carbon.

Compounds of Formulas I, II, and III have a chiral center at the carbon bearing the hydroxy radical. Preparation of an enantiomer is accomplished by starting with an appropriate optically active epoxide such as that of epichlorhydrin or glycidyl tosylate and the like and preparing the (R) and (S)-4-(N-R)-wherein N-R is an amino radical and (R) and (S) are the enantiomer designations -3-hydroxybutyronitrile which is reductively cyclized to give the (R) and (S)-3-pyrrolidinol.

A preferred aspect of the present invention is the reductive cyclization with hydrogen over catalyst comprised of Raney nickel of compounds of Formula II wherein Q is R- $\underset{H}{N}$ -

and R is methyl or ethyl and the compound produced is a pyrrolidinol of Formula Ia or Formula Ib having the structures:

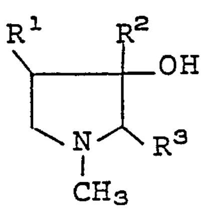

Ia

or

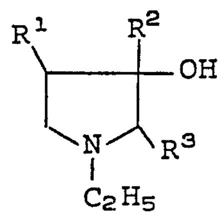

Ib

Two most preferred aspects of the invention is the reductive cyclization with hydrogen in the presence of catalyst comprised of Raney nickel of the compounds 4-(N-methyl)-3-hydroxybutyronitrile and 4-(N-ethyl)-3-hydroxybutyronitrile to give N-methyl-3-pyrrolidinol and N-ethyl-3-pyrrolidinol.

Starting compounds of Formula II wherein Q is $N_3$ maybe prepared by the known method represented by the following equations shown illustratively for compounds wherein $R^1$, $R^2$, and $R^3$ are all hydrogen,but the method is not limited to hydrogen radicals:

$ClCH_2CHOHCH_2Cl + NaCN \rightarrow ClCH_2CHOHCH_2CN$,

G. Braun, J. Amer. Chem. Soc. 52, 3167

$ClCH_2CHOHCH_2CN + NaN_3 \rightarrow N_3CH_2CHOHCH_2CN$ IIc

Starting compound of Formula II wherein Q is $NH_2$-are available by the known method represented by the following equation also shown illustratively for $R^1$, $R^2$, and $R^3$ = hydrogen, but the method is not limited to hydrogen radicals:

$N_3CH_2CHOHCH_2CN \xrightarrow{\text{reduce}} H_2NCH_2CHOHCH_2CN$ IIb

Intermediate chemical compounds of Formula IIa may be prepared by the reaction sequence outlined in Chart II.

CHART II

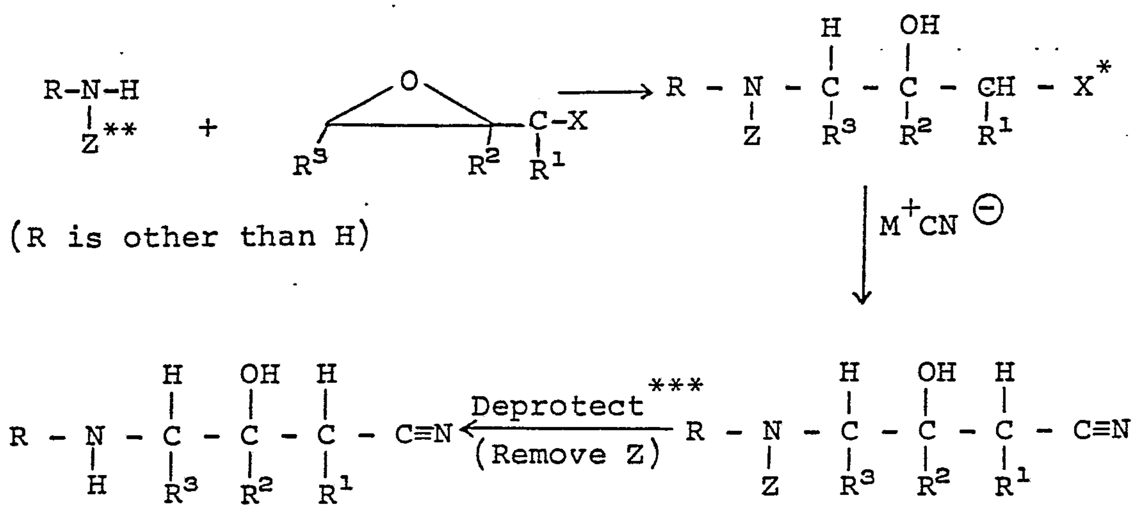


IIa

III

Footnotes to Chart II:

*X is any reactive leaving group such as a halogen atom -O-tosyl or -O-mesyl.

**Z is an amine protecting group, e.g., benzyl, diphenylmethyl, α-methylbenzyl, benzyloxycarbonyl, diphenylmethoxycarbonyl, β,β,β-trichloroethoxycarbonyl, t-butoxycarbonyl and isobutoxycarbonyl;

***benzyl, diphenylmethyl and α-methylbenzyl radicals are removed by hydrogenation over palladium on carbon in the presence of acidic protic solvent; benzyloxycarbonyl and t-butoxycarbonyl radicals are removed with acid; and the trichloroacetoxycarbonyl radical is removed with zinc and acetic acid. Compounds wherein R is benzyl may be obtained, for example, from a compound of Formula III wherein R is benzyl and Z is β,β,β-trichloroethoxycarbonyl and the deprotecting agent is zinc + acetic acid which leaves benzyl intact.

Selected alternative methods for preparing compounds of Formula II are illustrated schematically in Chart III.

## CHART III

Alternate Method A:

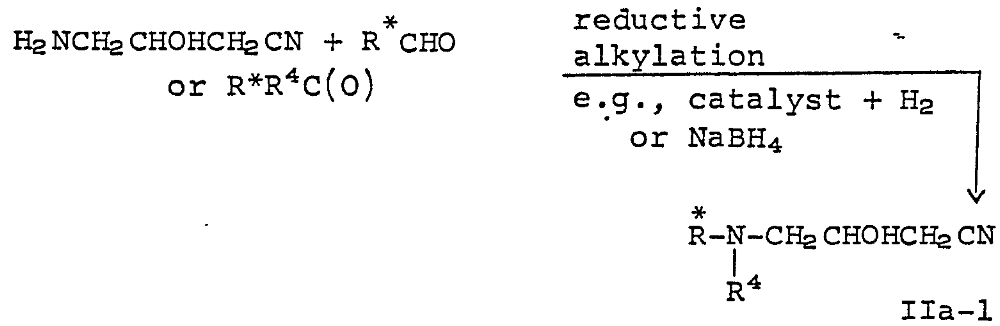


IIa-1

Alternate Method B:

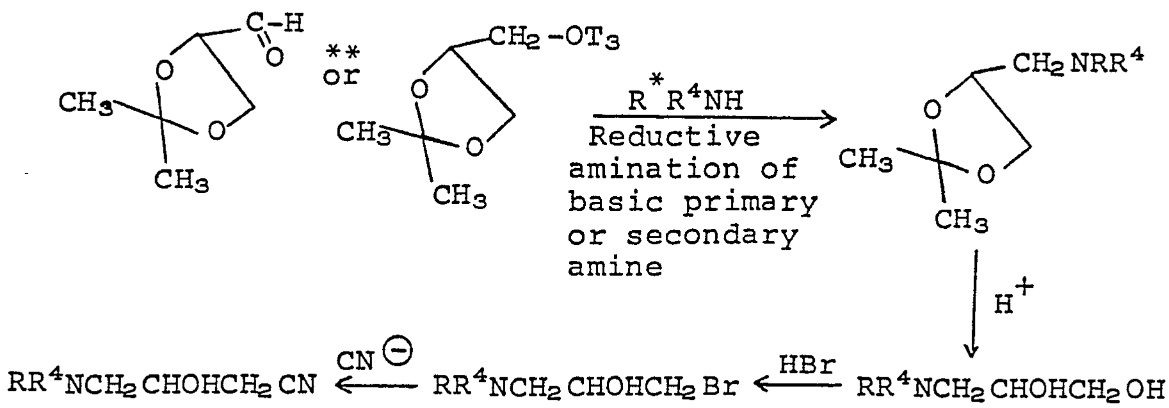


Alternate Method C:

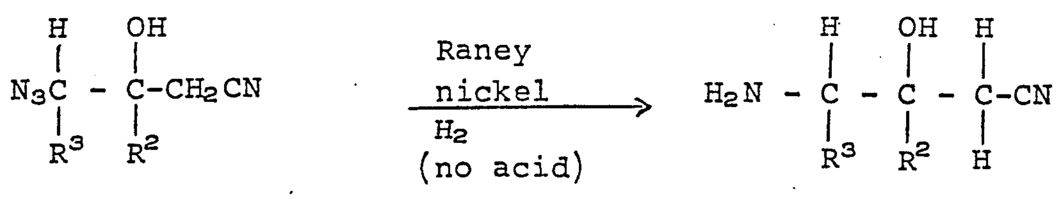


(cont.)

Chart III (cont.)

Alternate Method D:

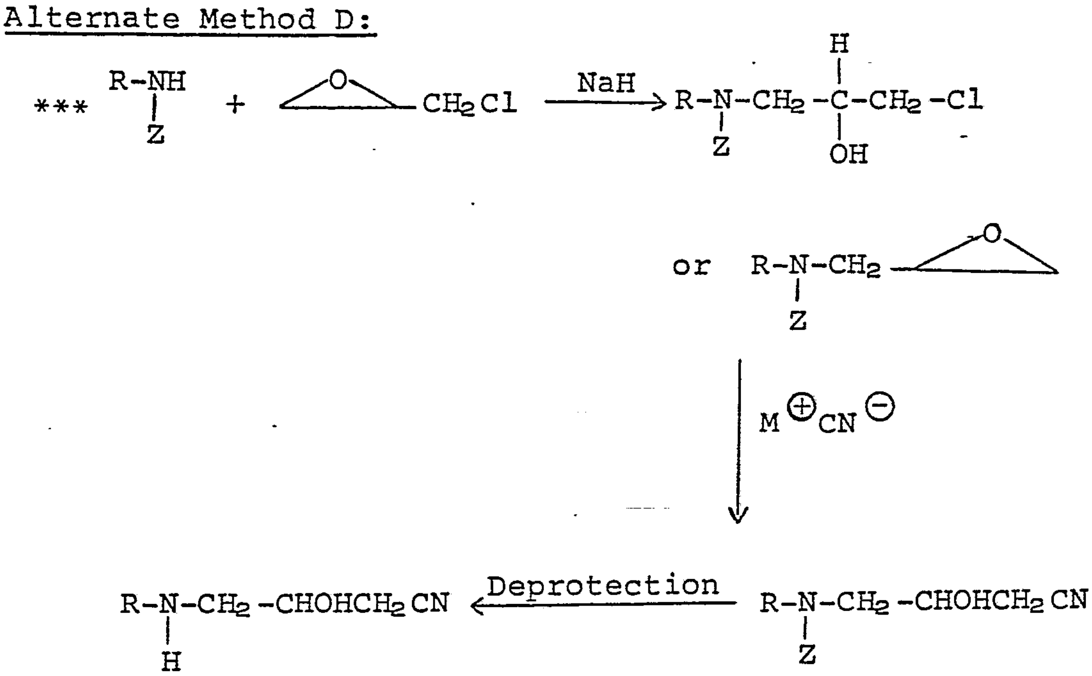


Footnotes to Chart III:

*R is as defined under Formula I.

**These starting compounds may be prepared by the method of Jung, M.E. et al., J. Amer. Chem. Soc. 102, 6304(1980).

***R is as defined under Formula I.

Z is selected from $-C(O)OCH_2\emptyset$

$-C(O)OCH_2CCl_3$

$-C(O)O$-t-Butyl

Epixode starting reactants of Chart II are available commecially or can be made by methods known in the art. A particularly versatile method is epoxidation of an appropriate allylic alcohol employing titanium tetraisopropoxide. See[The Merck Index 10th Ed (1983), page ONR-83] followed by displacement of the hydrogen of the hydroxyl group with a tosyl group. If desired, the resulting O-tosyl group may be replaced by a halo radical by reacting with an alkali-metal halo salt.

Starting epoxides wherein $R^1$ or $R^2$ or $R^3$ are methyl are known compounds and base catalyzed isomerism of these compounds has been studied, J. HETEROCYCLIC CHEMISTRY 6 (1969) pp 651-654. The compounds are:

Epichlorohydrin,

1-chloro-2,3-epoxy-2-methylpropane,

1-chloro-2,3-epoxybutane, and

3-chloro-1,2-epoxybutane

The method of preparation of (DL)-4-azido-3-hydroxybutyronitrile and (R)-4-azido-3-hydroxybutyronitrile shown by Jung, M. E. and Shaw, T. J., in J. AM. CHEM. SOC. (1980) 102 , pp 6304-6311 illustrates preparation of starting azido compounds. In that reference d1-4-tosyloxy-3-hydroxybutanenitrile or (R)-4-mesyloxy-3-hydroxybutanenitrile are reacted with potassium azide in acetonitrile.

The following intermediates, examples, and the preceding description and charts serve to illustrate the preparation of hydroxybutyronitriles which may be used as chemicals intermediates and the process for the

preparation of pyrrolidinols of the invention; however, the scope of the invention is not limited thereto.

Intermediate 1

4-(N-Methyl-N-benzylamino)-3-hydroxybutyronitrile.

To a stirred, cooled solution of 79 ml (1.0 mole) of epichlorohydrin in 600 ml of 200 proof ethanol was added 133 ml (1.0 mole) of N-(methyl)benzylamine, keeping the reaction mixture at 3 to 5°C. Addition time was 18 min. The temperature of the reaction mixture was allowed to rise to 29°C. over a 2 hr period and then raised to 37°C. briefly. Proton NMR analysis indicated about 10% of the N-methylbenzylamine was unreacted. An additional 7.9 ml (0.1 mole) of epichlorohydrin was added and the temperature was raised to 37°C. briefly. One hour later, 65 g (1.3 mole) of sodium cyanide in 150 ml of water was added over a 2 min period at 32°C. The reaction mixture was stirred overnight without heating or cooling. The mixture was heated to reflux for one hour and concentrated. The concentrate was diluted with methylene chloride and water. The organic layer was separated and washed once with sodium chloride solution. The aqueous layers were back extracted with a fresh portion of methylene chloride. The organic layers were combined, dried, filtered and evaporated to give 224.9 g of dark brown oil. Proton NMR showed mainly the title compound was present with some solvent. ($DCCl_3$, ppm): 7.35 (singlet, phenyl protons), 4.15 to 3.35 (multiplet, methine proton on carbon 3, hydroxyl proton on oxygen and the benzylic protons), 2.65 to 2.20 (multiplet, methylene protons on carbons 2 and 4 and the methyl protons on the amino group).

Intermediate 2

4-(N-Ethyl-N-benzylamino)-3-hydroxybutyronitrile.

In a procedure similar to Example 1, the title compound was prepared in 94% yield from 1 mole of N-ethylbenzylamine. Proton NMR analysis showed mainly product present with some solvent. ($DCCl_3$, ppm): 7.35 (singlet, phenyl protons), 4.10 to 3.40 (multiplet, methine proton on carbon 3, hydroxyl proton on oxygen, and the benzylic portons) 2.80 to 2.30 (multiplet methylene protons on carbons 2 and 4 and methylene protons on the ethyl group), 1.10 (triplet, methyl protons on the ethyl group).

Intermediate 3

4-Methylamino-3-hydroxybutyronitrile hydrochloride [1:1].

4-(N-Methyl-N-benzylamino)-3-hydroxybutyronitrile, 5.1 g (0.02 mole) was dissolved in 40 ml of isopropanol and 0.5 g of 5% palladium on carbon was added to the solution. The mixture was subjected to hydrogen gas under about 55 psi for 18 hr at 60°C. Mass spectrometry of a sample taken from the mixture indicated no reduction had occurred. About 2.1 ml (ca. 0.02 mole] of 37% hydrochloric acid and 0.5 g of 5% palladium on carbon were added and the mixture was subjected to hydrogen gas under 50 psi for 18 hr at room temperature. Approximately 0.02 molar equivalents of hydrogen was absorbed. A sample taken for mass spectroscopy showed product (m/e 115) and a small amount of starting material (m/e 205)unreacted. The mixture was filtered and the filtrate was concentrated to a light yellow liquid weighing 4.73 g. Proton NMR analysis showed mainly 4-methylamino-3-hydroxybutyronitrile and a small amount of starting benzyl compound and solvent was present, NMR analysis is as follows: ($D_2O$, ppm): 4.40 (multiplet, methine proton on carbon 3), 3.20 (multiplet, methylene protons on carbon 4 bearing the protonated amino groups), 2.80 (multiplet, methylene protons on carbon 2 and methyl protons on the amino group).

Intermediate 4

4-Azido-3-hydroxybutyronitrile.

A mixture of 48 g (0.40 mole) of 4-chloro-3-hydroxybutyronitrile, 52 g (0.080 mole) of sodium azide, 4.8 g of tetra-n-butylammonium bromide, 250 ml of chloroform and 100 ml of water was stirred while heating under reflux for 32 hr. The organic layer was separated and set aside. The aqueous layer was saturated with 4.8 g of potassium carbonate and then extracted three times with methylene chloride. The methylene chloride extracts were combined with the above organic layer and the solution was dried over anhydrous sodium sulfate and the mixture was filtered. The filtrate was evaporated to give a brown oil weighing 49.46 g, a 98% yield based on the crude. Proton NMR showed signals for the desired product and a small amount of tetra-n-butylammonium salt: ($CDCl_3$, ppm): 7.20 (singlet, chloroform), 4.35 to 3.90 (pentet, methine proton of product), 3.85 (singlet, hydroxyl proton of product), 3.45 (doublet, methylene protons next to the azide group of the product), 2.60 (doublet, methylene protons next to the cyano group of the product), 3.25 to 2.85 and 1.90 to 0.80 (multiplets, signals from about 4% of tetra-n-butylammonium salt).

Intermediate 5

4-Amino-3-hydroxy-butyronitrile.

To 22.5 g (0.178 mole) of 4-azido-3-hydroxy-butyronitrile (prepared in Intermediate 4) dissolved in 130 ml of isopropanol and 13 ml of water was added 2.5 g of Raney nickel catalyst. The mixture was hydrogenated at room temperature on a Parr® hydrogenator. A sample drawn at 2 hr reaction time showed by Mass spectrum analysis both starting material (m/e 127) and 4-amino-3-hydroxy-butyronitrile (m/e 101). Another sample taken at 4 hr reaction time showed by Mass spectrum analysis mainly(m/e 101) and a trace of(m/e 127).

Example 1

N-Ethyl-3-pyrrolidinol.
(via 4-(N-ethylamino)-3-hydroxybutyronitrile)

4-(N-Ethyl-N-benzylamino)-3-hydroxybutyronitrile, 109 g (0.50 mole), was dissolved in isopropanol and water. The solution was cooled and mixed with 41.6 ml (0.50 mole) of 37% hydrochloric acid. Catalyst (11 g, 5% palladium on carbon) was wetted with water and rinsed into the mixture with isopropanol. Total volumes of isopropanol and water were 800 ml and 80 ml respectively. The mixture was subjected to hydrogen under about 50 psi until absorption of hydrogen ceased, (0.50 mole). Mass spectroscopy of a sample indicated all starting compound had debenzylated, giving 4-(N-ethylamino)-3-hydroxybutyronitrile in the mixture. Raney nickel, 11 g (rinsed with water twice), was added to the mixture. This mixture was subjected to 50 psi hydrogen pressure at room temperature until absorption of hydrogen ceased. The mixture was filtered and the filtrate was concentrated to give light brown oil. To the oil was added 75 ml of polyethylene glycol 400, 32 g (0.4 mole) of 50% sodium hydroxide solution, 27.6 g (0.2 mole) of potassium carbonate and some methanol used in rinsing. The mixture was stirred, concentrated and then vacuum distilled to give 31.0 g (54%) of main fraction, distilling at 1 to 5 mm Hg and 65 to 75°C., the title compound. Analysis by proton NMR ($D_2O$, ppm) obtained was as follows: 4.80 (singlet, HOD), 4.40 (multiplet, methine proton), 3.10 to 1.30 (multiplets, all the methylene protons), 1.10 (triplet, methyl protons of the ethyl group).

Example 2

N-Methyl-3-pyrrolidinol.
(via 4-(N-methylamino)-3-hydroxy-butyronitrile)

4-(N-Methyl-N-benzylamino)-3-hydroxybutyronitrile was debenzylated to 4-(N-methylamino)-3-hydroxy-butyronitrile which was converted to the title compound in 56% overall yield by the procedure of Example

1. The product distilled at 1-5 mm Hg and 40 to 50°C. Analysis by proton NMR ($D_2O$, ppm) obtained was as follows: 4.80 (singlet, HOD), 4.40 (multiplet, methine proton), 3.00 to 1.30 (multiplets and a singlet, the singlet is the methyl protons of the N-methyl group. The multiplets are the methylene protons). The foregoing spectrum matches with the spectrum of an authentic sample of N-methyl-3-pyrrolidinol.

Example 3

3-Pyrrolidinol.
(via 4-amino-3-hydroxybutyronitrile)

A solution of 22.5 g (0.178 mole) of 4-azido-3-hydroxy-butyronitrile in 130 ml of isopropanol and 13 ml of water together with 2.5 g Raney nickel was subjected to hydrogen at room temperature in a Parr hydrogenator for 4 hr. Mass spectrum analysis indicated only a trace of starting material m/e 127 was unreacted, the main product exhibiting m/e 101, that of 4-amino-3-hydroxybutyronitrile.

The mixture was made slightly acidic by adding 13.6 ml of 37% hydrochloric acid, and 1.0 g of additional Raney nickel was added. The mixture was subjected to 50 psi hydrogen pressure overnight, without hydrogen uptake occurring. The mixture was filtered and 2.5 g of fresh Raney nickel was added to the filtrate. The mixture was hydrogenated at room temperature and about 50 psi; hydrogen uptake occurred and sampling for mass spectrum analysis indicated the material present was mainly 3-pyrrolidinol (m/e 88). The catalyst was removed by filtration and the filtrate was evaporated to give a brown oil. To the oil were added 30 ml of polyethyleneglycol-400, 8 g (0.1 mole) of 50% aqueous sodium hydroxide solution, 11 g (0.08 mole) of potassium carbonate and some methanol for rinsing. The resultant mixture was stirred for a period of time to assure acid neutralization and then concentrated on a rotary evaporator. The concentrate was vacuum distilled. The desired product, 3-pyrrolidinol, was collected from the still at 80-120°C. under 5 to 20 mm Hg pressure. Weight of product liquid obtained was 4.7 g (30% yield). Proton NMR ($D_2O$, ppm) analysis obtained was as follows: 4.85 (singlet, HOD the hydroxyl and the amino proton), 4.55 to 4.25 (multiplet, methine proton) 3,85 to 2.50 (multiplet, methylene protons on carbons 2 and 5, both are next to the nitrogen) 2.30 to 1.40 (multiplet, methylene protons at carbon 4).

Example 4

(R)-N-Methyl-3-pyrrolidinol and (S)-N-Methyl-3-pyrrolidinol.

2-R-(+)-Glycidyl tosylate and 2-S-(-)-glycidyl tosylate available from the Aldrich Chemical Co., P. 0. Box 355 Milwaukee, Wisconsin 53201, USA, in 200 proof ethanol are separately reacted with N-(methyl)-benzyl amine as in Intermediate 1, and the products thereof are each reacted with sodium cyanide as in Intermediate 1 and isolated as the optically active (R) and (S)-4-(N-methyl-N-benzylamino3-hydroxybutyronitriles. These nitriles are hydrogenated over palladium on carbon in acidic isopropanol and water solution and the resulting secondary 4-methylamino-3-hydroxybutyronitriles reductively cyclized by hydrogenation over Raney nickel as in Example 1 to give the title compounds.

Example 5

(R)-N-Methyl-3-pyrrolidinol.

(S)-Epichlorohydrin is reacted with N-(methyl)benzyl amine and the product thereof is reacted with sodium cyanide as in Intermediate 1 and isolated as the optically active R-4-(N-methyl-N-benzylamino)-3-hydroxybutyronitrile. This nitrile is hydrogenated over palladium on carbon in acidic isopropanol and water solution and the resulting 4-methylamino-3-hydroxybutyronitrile is reductively cyclized by hydrogenation over Raney nickel to give the title compound.

Example 6

N,4-Dimethyl-3-pyrrolidinol and N,2-Dimethyl-3-pyrrolidinol

Following the procedure of Intermediate 1, 2,3-epoxybutyl chloride is reacted with N-(methyl)-benzylamine. Sodium cyanide is added. The products of these reactions are expected to be

4-[N-(methyl)benzylamino]-4-methyl-3-hydroxy-butyronitrile, and

4-[N-(methyl)benzylamino]-2-methyl-3-hydroxy butyronitrile.

When these nitriles are deprotected to remove the benzyl radical by hydrogenation over palladium on carbon and the products thereof are reductively cyclized with hydrogen over Raney nickel, the title compounds are obtained. When the products are present in a mixture, they are separated by Spinning Band Column Distillation.

Example 7

N,3-Dimethyl-3-pyrrolidinol.

Following the procedure of Example 2 and substituting 4-(N-methylamino)-3-hydroxy-3-methyl-butyronitrile for 4-(N-methylamino)-3-hydroxybutyronitrile, the title compound is obtained.

Example 8

N,4-Dimethyl-3-pyrrolidinol.

Following the procedure of Example 2 and substituting 4-(N-methylamino)-3-hydroxy-2-methyl-butyronitrile for 4-(N-methylamino)-3-hydroxybutyronitrile, the title compound is obtained.

## Claims

1. A process for the preparation of a pyrrolidinol compound having a general formula I or a salt thereof, characterised in that the process comprises reductive cyclisation of a hydroxybutyronitrile compound having a general formula II or a salt thereof, in an acid containing solution, with hydrogen gas, in the presence of a catalyst comprising Raney nickel:

Formula I

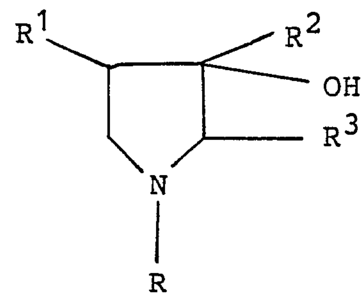

Formula II

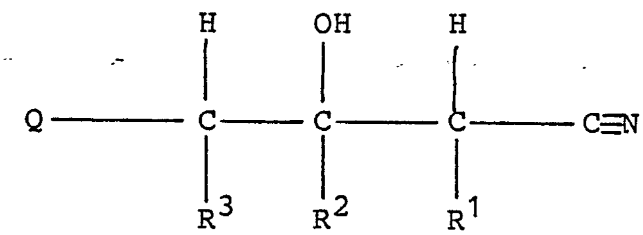

where R represents
a hydrogen atom,
a lower alkyl group ($C_1$ to $C_8$)
a lower alkenyl group ($C_2$ to $C_8$)
a cycloalkyl group ($C_3$ to $C_9$)
a cycloalkyl-loweralkyl group ($C_4$ to $C_{13}$)
a phenyl-lower alkyl group ($C_7$ to $C_{14}$),
or
a $(Y)_{1-3}$-phenyl-lower alkyl group ($C_7$ to $C_{14}$);
and
Y represents
a lower alkyl group ($C_1$ to $C_8$),
a lower alkoxy group ($C_1$ to $C_8$),
a halogen atom
or
a trifluoromethyl group;
and each of $R^1$, $R^2$ and $R^3$ independently represents
a hydrogen atom,
a lower alkyl group ($C_1$ to $C_8$)
or
a lower alkenyl group ($C_2$ tp $C_8$);
and where Q represents a group R-(NH)-or an azido group.

2. A process according to claim 1 characterised in that the process further comprises separation of the catalyst from the pyrrolidinol compound which is in an acid containing solution.

3. A process according to claim 1 or claim 2 characterised in that the process further comprises substantial evaporation of a solvent from the acid containing solution, addition of a high boiling dilueunt, neutralisation of the acid addition salt with a base, and vacuum distillation to produce a distillate comprising the pyrrolidinol compound as a free base.

4. A process according to any one of the preceding claims characterised in that the catalyst comprises a mixture of a Raney nickel catalyst and a palladium on carbon catalyst.

5. A process according to any one of the preceding claims characterised in that the pyrrolidinol compound is substantially in either the 3 - R or the 3 - S enantiomeric form.

6. A process according to any one of the preceding claims characterised in that the hydroxybutyronitrile compound is:
4 - (N-ethylamino)-B-hydroxybutyronitrile
4 - (N-methylamino)-3-hydroxybutyronitrile
4 - amino-3-hydroxybutyronitrile
4 - azido-3-hydroxybutyronitrile
4 - methylamino-3-hydroxybutyronitrile
4 - ethylamino-3-hydroxybutyronitrile
4 - (N-methylamino)-3-hydroxy-3-methylbutyronitrile
or 4 - (N-methylamino)-3-hydroxy-2-methylbutyronitrile
or a salt thereof.

7. A hydroxybutyronitrile compound characterised in that the compound has the general formula IV or a salt thereof:

Formula IV

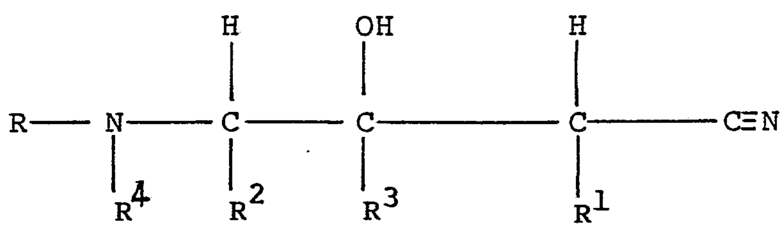

where R represents
a hydrogen atom
a lower alkyl group ($C_1$ to $C_8$)
a lower alkenyl group ($C_2$ to $C_8$)
a cycloalkyl group ($C_3$ to $C_9$)
a cycloalkyl-lower alkyl group ($C_4$ to $C_{13}$)
a phenyl-lower alkyl group ($C_7$ to $C_{14}$)
or
a $(Y)_{103}$-phenyl-lower alkyl group ($C_7$ to $C_{14}$)
and
Y represents
a lower alkyl group ($C_1$ to $C_8$)
a lower alkoxy group ($C_1$ to $C_8$)
a halogen atom
or
a trifluoromethyl group
and each of
$R^1$, $R^2$ and $R^3$ independently represents
a hydrogen
a lower alkyl group ($C_1$ to $C_8$)
or a lower alkenyl group ($C_2$ to $C_8$)
where $R^4$ represents a hydrogen atom or Z
and Z represents an amine protecting radical
and with the proviso that R represents a hydrogen atom only when $R^4$ represents Z, and Z represents a benzyl group.

8. A compound according to claim 7 characterised in that
Z represents
a benzyl group
an alpha-methylbenzyl group
a diphenylmethyl group
an alpha-phenylmethyl group
a benzyloxycarbonyl group

a diphenylmethoxycarbonyl group
a beta, beta, beta-trichloroethoxycarbonyl group
an isobutoxycarbonyl group
an phenylmethoxycarbonyl group
or
a t-butyloxycarbonyl group

9. A compound according to claim 7 or claim 8 characterised in that R does not represent a hydrogen atom.

10. A compound according to claim 9 characterised in that $R^4$ represents a hydrogen atom.

11. A compound according to claim 7, claim 8 or claim 9 characterised in that $R^4$ represents Z.

12. A compound characterised in that the compound is:
4 - (N-ethylamino)-B-hydroxybutyronitrile
4 - (N-methylamino)-3-hydroxybutyronitrile
4 - amino-3-hydroxybutyronitrile
4 - azido-3-hydroxybutyronitrile
4 - methylamino-3-hydroxybutyronitrile
4 - ethylamino-3-hydroxybutyronitrile
4 - (N-methylamino)-3-hydroxy-3-methyl-butyronitrile
or 4 - (N-methylamino)-3-hydroxy-2-methylbutyronitrile
or a salt thereof.

13. A process for the preparation of a compound having the general formula IV or a salt thereof

Formula IV

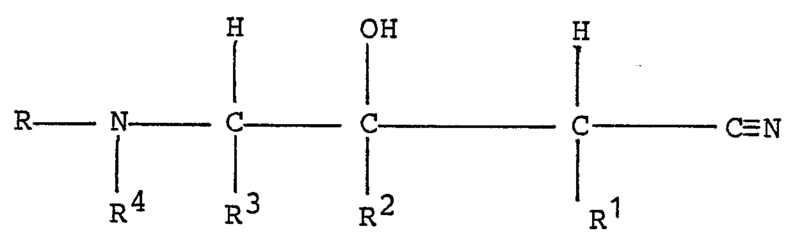

where R represents
a lower alkyl group ($C_1$ to $C_8$)
a lower alkenyl group ($C_2$ to $C_8$)
a cycloalkyl group ($C_3$ to $C_9$)
a cycloalkyl-lower alkyl group ($C_4$ to $C_{13}$)
a phenyl-lower alkyl group ($C_7$ to $C_{14}$)
or
a $(Y)_{1\text{-}3}$-phenyl-lower alkyl group ($C_7$ to $C_{14}$)
and
Y represents
a lower alkyl group ($C_1$ to $C_8$)
a lower alkoxy group ($C_1$ to $C_8$)
a halogen atom
or
a trifluoromethyl group
and each of
$R^1$, $R^2$ and $R^3$ independently represents
a hydrogen atom
a lower alkyl group ($C_1$ to $C_8$)
or a lower alkenyl group ($C_2$ to $C_8$)
$R^4$ represents a hydrogen atom or Z
and Z represents an amine protecting radical
with the proviso that R represents a hydrogen atom only when Z represents a benzyl group characterised in that the process comprises reaction of a compound having the formula VII with a metal cyanide:-

Formula VII

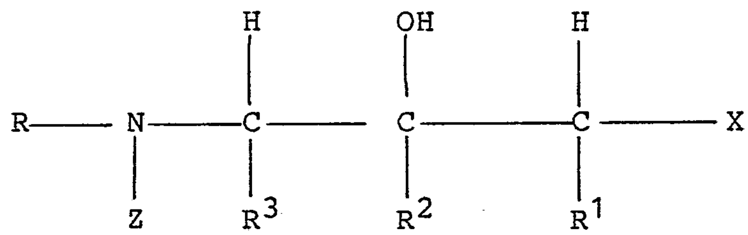

wherein X represents a reactive leaving group and $R^1$, $R^2$, $R^3$ and Z are as defined for general formula IV.

14. A process according to claim 11 where $R^4$ represents a hydrogen atom, characterised in that the process further comprises deprotecting the amine group.

Claims for the following Contracting State: ES

1. A process for the preparation of a pyrrolidinol compound having a general formula I or a salt thereof, characterised in that the process comprises reductive cyclisation of a hydroxybutyronitrile compound having a general formula II or a salt thereof, in an acid containing solution, with hydrogen gas, in the presence of a catalyst comprising Raney nickel:

Formula I

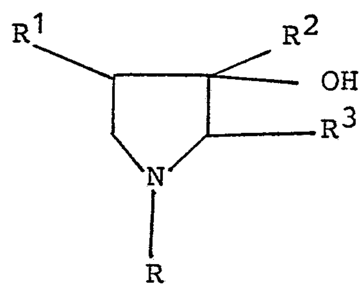

Formula II

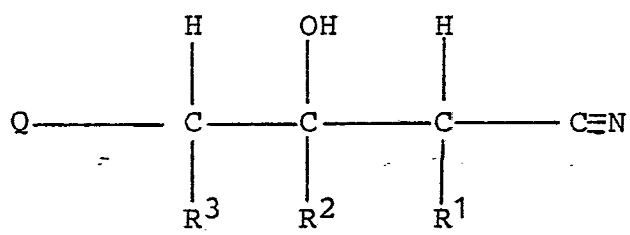

where R represents
a hydrogen atom,
a lower alkyl group ($C_1$ to $C_8$)
a lower alkenyl group ($C_2$ to $C_8$)
a cycloalkyl group ($C_3$ to $C_9$)
a cycloalkyl-loweralkyl group ($C_4$ to $C_{13}$)
a phenyl-lower alkyl group ($C_7$ to $C_{14}$),
or
a $(Y)_{1-3}$-phenyl-lower alkyl group ($C_7$ to $C_{14}$);
and

Y represents
a lower alkyl group ($C_1$ to $C_8$),
a lower alkoxy group ($C_1$ to $C_8$),
a halogen atom
or
a trifluoromethyl group;
and each of $R^1$, $R^2$ and $R^3$ independently represents
a hydrogen atom,
a lower alkyl group ($C_1$ to $C_8$)
or
a lower alkenyl group ($C_2$ to $C_8$);
and where Q represents a group R-(NH)-or an azido group.

2. A process according to claim 1 characterised in that the process further comprises separation of the catalyst from the pyrrolidinol compound which is in an acid containing solution.

3. A process according to claim 1 or claim 2 characterised in that the process further comprises substantial evaporation of a solvent from the acid containing solution, addition of a high boiling dilueunt, neutralisation of the acid addition salt with a base, and vacuum distillation to produce a distillate comprising the pyrrolidinol compound as a free base.

4. A process according to any one of the preceding claims characterised in that the catalyst comprises a mixture of a Raney nickel catalyst and a palladium on carbon catalyst.

5. A process according to any one of the preceding claims characterised in that the pyrrolidinol compound is substantially in either the 3 - R or the 3 - S enantiomeric form.

6. A process according to any one of the preceding claims characterised in that the acid containing solution comprises a non-interfering acid.

7. A process according to any one of the preceding claims characterised in that the hydroxybutyronitrile compound is:
4 - (N-ethylamino)-B-hydroxybutyronitrile
4 - (N-methylamino)-3-hydroxybutyronitrile
4 - amino-3-hydroxybutyronitrile
4 - azido-3-hydroxybutyronitrile
4 - methylamino-3-hydroxybutyronitrile
4 - ethylamino-3-hydroxybutyronitrile
4 - (N-methylamino)-3-hydroxy-3-methyl-butyronitrile
or 4 - (N-methylamino)-3-hydroxy-2-methyl-butyronitrile
or a salt thereof.

8. A process according to any one of the preceding claims characterised in that the hydroxybutyronitrile compound comprises the following or salts thereof:
4-(N-ethylamino)-3-hydroxybutyronitrile [for the preparation of N-ethyl-3-pyrrolidinol],
4-(N-methylamino)-3-hydroxybutyronitrile [for the preparation of N-methyl-3-pyrrolidinol],
4-amino-3-hydroxybutyronitrile [for the preparation of 3-pyrrolidinol],
4-azido-3-hydroxybutyronitrile [for the preparation of 3-pyrrolidinol],
4-methylamino-3-hydroxybutyronitrile [for the preparation of N-methyl-3-pyrrolidinol ],
4-ethylamino-3-hydroxybutyronitrile [for the preparation of N-ethyl-3-pyrrolidinol],
4-(N-methylamino)-3-hydroxy-3-methyl-butyronitrile [for the preparation of N,3-Dimethyl-3-pyrrolidinol],
4-(N-methylamino)-3-hydroxy-2-methyl-butyronitrile [for the preparation of N,4-Dimethyl-3-pyrrolidinol],
or their salts.

9. A process for the preparation of a compound having the general formula IV or a salt thereof:

Formula IV

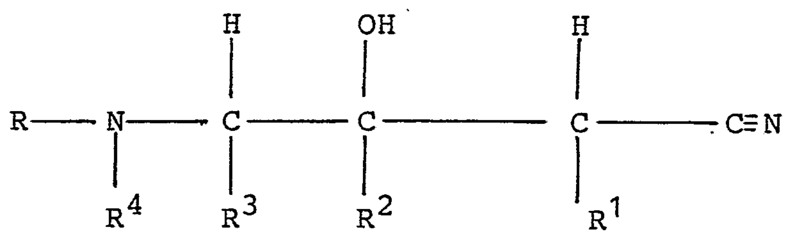

where R represents a lower alkyl group ($C_1$ to $C_8$) a lower alkenyl group ($C_2$ to $C_8$) a cycloalkyl group ($C_3$ to $C_9$) a cycloalkyl - lower alkyl group ($C_4$ to $C_{13}$) a phenyl-lower alkyl group ($C_7$ to $C_{14}$) or a $(Y)_{1-3}$-phenyl-lower alkyl group ($C_7$ to $C_{14}$) and Y represents a lower alkyl group ($C_1$ to $C_8$) a lower alkoxy group ($C_1$ to $C_8$) a halogen atom or a trifluoromethyl group and each of $R^1$, $R^2$ and $R^3$ independently represents a hydrogen atom a lower alkyl group ($C_1$ to $C_8$) or a lower alkenyl group ($C_2$ to $C_8$) $R^4$ represents a hydrogen atom or Z and Z represents an amine protecting radical with the proviso that R represents a hydrogen atom only when Z represents a benzyl group, characterised in that the process comprises reaction of a compound of formula VII with a metal cyanide.

Formula VII

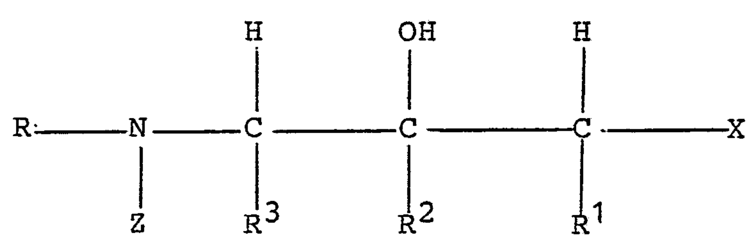

wherein X represents a reactive leaving group and $R^1$, $R^2$, $R^3$ and Z are as defined for general formula IV.

10. A process according to claim 9 characterised in that X represents a halogen atom an -o-tosyl group or an -o-mesyl group

11. A process according to claim 9 where $R^4$ represents a hydrogen atom, characterised in that the process further comprises deprotecting the amine group.

12. A process according to claim 9 or claim 11 characterised in that the compound is
4 - (N-ethylamino)-B-hydroxybutyronitrile
4 - (N-methylamino)-3-hydroxybutyronitrile
4 - amino-3-hydroxybutyronitrile
4 - azido-3-hydroxybutyronitrile
4 - methylamino-3-hydroxybutyronitrile
4 - ethylamino-3-hydroxybutyronitrile
4 - (N-methylamino)-3-hydroxy-3-methyl-butyronitrile
or 4 - (N-methylamino)-3-hydroxy-2-methyl-butyronitrile
or a salt thereof.